(19) Europäisches Patentamt / European Patent Office / Office européen des brevets

(11) **EP 3 928 627 A1**

(12) **EUROPEAN PATENT APPLICATION**
published in accordance with Art. 153(4) EPC

(43) Date of publication:
**29.12.2021 Bulletin 2021/52**

(21) Application number: **20759686.7**

(22) Date of filing: **19.02.2020**

(51) Int Cl.:
*A23C 11/10* (2021.01)   *A23L 25/00* (2016.01)
*C12N 9/10* (2006.01)

(86) International application number:
**PCT/JP2020/006432**

(87) International publication number:
**WO 2020/171105 (27.08.2020 Gazette 2020/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.02.2019 JP 2019029904**

(71) Applicants:
• **Amano Enzyme Inc.**
**Nagoya-shi**
**Aichi 460-8630 (JP)**

• **Amano Enzyme Europe Ltd.**
**Oxfordshire, OX75SR (GB)**

(72) Inventor: **FUJIOKA, Hiroki**
**Norton, Oxfordshire  OX75SR (GB)**

(74) Representative: **Müller-Boré & Partner**
**Patentanwälte PartG mbB**
**Friedenheimer Brücke 21**
**80639 München (DE)**

(54) **PREVENTION OF COAGULATION OF NUT MILK**

(57)    The present invention addresses the problem of creating an effective means for preventing the aggregation in a nut milk. The dispersibility of a nut milk is increased by treating the same with a protein deamidase. Thus, when the nut milk is added to a liquid beverage, a liquid food, etc., the aggregation in the nut milk is prevented.

EP 3 928 627 A1

**Description**

TECHNICAL FIELD

The present invention relates to nut milk. The invention relates specifically to nut milk with improved dispersibility (hard-to-aggregate nut milk) and use thereof. The present application claims the priority of Japanese Patent Application No. 2019-029904, filed on February 21, 2019, the entire content of which is herein incorporated by reference.

BACKGROUND ART

On the background of, for instance, allergy problems, an increase in the number of vegetarians, religious reasons, proteins derived from soybeans, which are a plant origin, have become widespread as an alternative raw material for foods or beverages using an animal-derived milk protein source exemplified by cow milk. However, as widespread use progresses, it has been found that soybean also causes allergy, and in recent years, plant-derived protein raw materials replacing soybean have increasingly developed. In fact, proteins derived from cereals such as peas, rice, or oats and proteins derived from nuts such as almonds, cashew nuts, or peanuts have been commercialized as foods and/or beverages one after another as substitutes for soybeans. Thus, it can be said that to avoid allergy, there are high needs and demands for diversification of protein raw materials derived from plants other than soybeans.

Meanwhile, in the case of replacing the milk protein raw materials with plant-derived protein raw materials, there are some cases where the milk protein raw materials cannot be replaced as they are because the protein type and functionality or aroma or taste components are different. For example, it has been known that aggregation is caused by adding nut milk, such as almond milk or peanut milk, as a substitute for cow milk, to an acidic beverage such as coffee or black tea. Usually, such aggregation does not occur in cow milk, and can be said to be a nut milk-specific phenomenon.

As far as the present inventors know, no report (e.g., any literature) has clearly demonstrated the mechanism of protein aggregation occurring when nut milk is added to an acidic liquid food and any countermeasures therefor. Although trial and error of countermeasures (e.g., mixing after minimizing the temperature difference between nut milk and coffee, slowly pouring coffee into nut milk) among consumers are recognized, a radical solution has yet to be provided.

On the other hand, it has been known that the dispersion stability of milk proteins is unstable under acidic conditions at or near the isoelectric point, and, for instance, precipitation and/or aggregation are likely to occur in acidic milk beverages. In order to prevent this milk protein aggregation, for example, polysaccharides such as pectin and/or carboxymethylcellulose are added (see, for example, Patent Documents 1 and 2). Although use of such a dispersion stabilizer may prevent aggregation in nut proteins, it is essential to use an additive. In addition, in the case of using polysaccharides, a side effect such as an increase in viscosity may occur depending on the additive amount.

A protein deamidase-treated coffee whitener has been disclosed for preventing milk protein aggregation without adding any additive (Patent Document 3). However, the coffee whitener is an emulsifier-containing product, and its use is limited to those using whiteners, such as coffee or black tea.

PRIOR ART DOCUMENTS

PATENT DOCUMENTS

Patent Document 1: WO 2012/176852 A
Patent Document 2: Japanese Patent No. 3885194
Patent Document 3: WO 2011/108633 A

SUMMARY OF THE INVENTION

PROBLEMS TO BE SOLVED BY THE INVENTION

The aggregation phenomenon, which seems to be unique to nut milk, lowers the value (e.g., use value, product value) of nut milk, the demand and application of which are expected to further increase in the future. Thus, in order to increase the value of nut milk and promote its use or application, the present invention addresses the problem of creating an effective means for preventing aggregation in nut milk, and particularly providing nut milk that is unlikely to aggregate during use in liquid beverages (in particular, acidic liquid beverages) and/or liquid foods (in particular, acidic liquid foods) without adding any additive.

MEANS FOR SOLVING THE PROBLEM

The present inventors have conducted intensive research in light of the above problem and have focused on deamidation of proteins, and have attempted to improve the dispersibility of nut milk by treating the nut milk with a protein deamidase. Note that there has been no report so far about an example of use of a protein deamidase in nut milk.

First, regarding the addition of nut milk to coffee, which can be said to be one of typical applications, whether or not treatment with a protein deamidase is effective in preventing aggregation was checked. Surprisingly, use of enzyme-treated almond milk did not cause protein aggregation. Based on this finding, specific experiments assuming various uses were conducted. As a result, it has become clear that treatment with a protein deamidase is markedly effective for improving the dispersibility of nut milk in general. In other words, an effective means for preventing aggregation in nut milk has been found. This has resulted in successful preparation of hard-to- aggregate nut milk having improved dispersibility without using any additive such as an emulsifier. In addition, many useful findings when nut milk is used for various beverages or foods have been obtained. Based on these results, the following items of the invention are provided. Note that as described above, use of a protein deamidase for improving the dispersibility of a coffee whitener has been disclosed. The coffee whitener is generally prepared by homogenizing, with a homogenizer having excellent shear force (e.g., a high-pressure homogenizer), a liquid to be emulsified containing edible fat and oil as a main raw material and having an emulsifier, and if necessary, for instance, a milk component(s), a thickener, or a flavoring agent added. The raw materials, composition, and preparation procedure, for instance, are totally different from those of nut milk. Thus, not only the effectiveness of the means effective in improving the dispersibility of the coffee whitener over the nut milk but also the possibility of its application cannot be predicted at all.

[1] Nut milk having been treated with a protein deamidase.

[2] The nut milk according to [1], wherein a raw material nut is one or two or more nuts selected from almonds, cashew nuts, hazelnuts, pecan nuts, macadamia nuts, pistachios, walnuts, Brazil nuts, peanuts, coconuts, chestnuts, sesames, and pine nuts.

[3] The nut milk according to [1] or [2], wherein a nut protein concentration thereof is from 0.2% (w/v) to 10.0% (w/v).

[4] The nut milk according to any one of [1] to [3], wherein dispersibility thereof is improved by the treatment.

[5] The nut milk according to [4], wherein no protein aggregation occurs in a case of being mixed with a weakly acidic to weakly alkaline liquid, provided that a pH of mixture is 5 or higher.

[6] The nut milk according to [5], wherein the liquid has a pH of from 5 to 7.

[7] The nut milk according to [5], wherein the liquid is a beverage or liquid food selected from the group consisting of coffee, coffee beverages, tea, tea beverages, fruit juice, fruit juice beverages, sports drinks, nutritional drinks, soup, curry, cocoa beverages, and chocolate beverages.

[8] The nut milk according to any one of [1] to [7], wherein no emulsifier or polysaccharide thickener for preventing aggregation is included.

[9] The nut milk according to any one of [1] to [8], wherein the protein deamidase is an enzyme derived from a *Chryseobacterium* microorganism.

[10] The nut milk according to [9], wherein the *Chryseobacterium* microorganism is *Chryseobacterium proteolyticum.*

[11] A process for producing nut milk having improved dispersibility, comprising treating nut milk with a protein deamidase.

[12] The production process according to [11], comprising the following steps (1) and (2) of:

(1) providing nut milk; and
(2) treating the nut milk provided in step (1) with a protein deamidase.

[13] The production process according to [12], wherein the nut milk in step (1) is nut milk before heat sterilization.

[14] The production process according to [13], further comprising the following step (3) of:

(3) performing heat treatment.

[15] A beverage or liquid food comprising the nut milk according to any one of [1] to [10].

[16] The beverage or liquid food according to [15], wherein the beverage or liquid food has a pH of 5 or higher.

[17] The beverage or liquid food according to [15], wherein the beverage or liquid food is selected from the group consisting of coffee beverages, coffee whiteners, tea beverages, fruit juice beverages, sports drinks, nutritional drinks, soups, curry, cocoa beverages, and chocolate beverages.

BRIEF DESCRIPTION OF THE DRAWINGS

Fig. 1 is a summary of experimental results (relationship between the protein concentration of nut milk and the aggregation/aggregation preventing effect).

Fig. 2 is a summary of experimental results (relationship between the pH of liquid and the aggregation/aggregation preventing effect).

Fig. 3 is a summary of experimental results (the effect of preventing aggregation in various liquids). Note that the results of Experiment 1 (prevention of aggregation in coffee) are also shown.

Fig. 4 is a summary of experimental results (the effect of preventing aggregation in nut milk in addition to almond milk).

Fig. 5 is a summary of experimental results (relationship between the temperature of liquid and the aggregation/aggregation preventing effect).

Fig. 6 is a summary of experimental results (in which enzyme treatment conditions (the amount of enzyme added, the reaction temperature, and the reaction time) were examined).

EMBODIMENTS OF THE INVENTION

1. Nut Milk with Improved Dispersibility

A first aspect of the invention relates to nut milk having improved dispersibility (also referred to as a nut protein-containing beverage). The nut milk of the invention is treated with a protein deamidase, and as a result of the treatment, its dispersibility is improved. The nut milk of the invention exhibits excellent dispersibility, and is thus unlikely to cause aggregation when added to, for instance, a beverage such as coffee or black tea without using any dispersibility-improving additive (e.g., an emulsifier, a polysaccharide thickener (e.g., pectin, carboxymethyl cellulose), a salt). This property enables use for various beverages or foods.

Nut milk represented by almond milk is plant milk using nuts as a raw material, and is generally prepared by processes such as pulverization, immersion/dissolution, mixing/stirring, filtration, homogenization, and sterilization of de-nucleated nuts. The procedure for preparing nut milk used in the invention is not particularly limited. Meanwhile, nut milk provided by a raw material manufacturer or commercially available nut milk may be purchased and used in the invention.

The nut milk of the invention is obtained by treating nut milk with a protein deamidase to improve the dispersibility thereof. Hereinafter, for convenience of description, the nut milk to be subjected to the treatment with a protein deamidase is referred to as "untreated nut milk".

The nuts which are a raw material for untreated nut milk are not particularly limited. Examples of the raw material nuts include almonds, cashew nuts, hazelnuts, pecan nuts, macadamia nuts, pistachios, walnuts, Brazil nuts, peanuts, coconuts, chestnuts, sesames, or pine nuts.

Untreated nut milk, in which two or more kinds of nuts are used in combination (e.g., a combination of almonds and cashew nuts or a combination of almonds and peanuts), can also be used.

The protein concentration in the untreated nut milk is not particularly limited, and the untreated nut milk used has a protein concentration of, for example, from 0.2% (w/v) to 10.0% (w/v), preferably from 0.2% (w/v) to 8.0% (w/v), and more preferably from 0.2% (w/v) to 5.0% (w/v). Note that the protein concentration of the nut milk after the protein deamidase treatment is likewise, for example, from 0.2% (w/v) to 10.0% (w/v), preferably from 0.2% (w/v) to 8.0% (w/v), and more preferably from 0.2% (w/v) to 5.0% (w/v).

The protein deamidase used in the invention directly acts on an amide group of a protein to perform deamidation without cleaving a peptide bond or crosslinking proteins. The type and/or origin of the enzyme, for instance, are not particularly limited as long as the enzyme exerts the action. Examples of the protein deamidase include: a protein deamidase derived from the genus *Chryseobacterium, Flavobacterium, Empedobacter, Sphingobacterium, Aureobacterium,* or *Myroides,* which are disclosed in, for instance, JP 2000-50887A, JP 2001-218590A, or WO 2006/075772; or a commercially available protein glutaminase derived from the genus *Chryseobacterium.* Preferably used is an enzyme derived from the genus *Chryseobacterium* (specific examples thereof include an enzyme derived from *Chryseobacterium proteolyticum* (e.g., a protein glutaminase "Amano" 500, manufactured by Amano Enzyme Inc.)).

The protein deamidase used may be one prepared from culture broth of microorganism that produces the protein deamidase. The microorganism used for the preparation of the protein deamidase is not particularly limited, and examples of the available microorganism that produces the enzyme include microorganisms belonging to the genus *Chryseobacterium, Flavobacterium, Empedobacter, Sphingobacterium, Aureobacterium,* or *Myroides.* Specific examples of microorganisms suitable for preparation of the protein deamidase include *Chryseobacterium sp.* No. 9670 belonging to the genus *Chryseobacterium.*

For example, the protein deamidase can be obtained from culture broth or bacterial cells of the above-mentioned microorganism. Specifically, secreted proteins can be recovered from the culture broth, and the other proteins can be recovered from the bacterial cells. As a method for preparing a protein deamidase from culture broth, a known protein separation and purification method (e.g., centrifugation, UF concentration, salting-out, various types of chromatography using an ion exchange resin) may be used. For example, the culture broth may be centrifuged to remove bacterial cells, and then salting-out and chromatography, for instance, are used in combination to obtain an enzyme of interest. In the case of recovering the enzyme from bacterial cells, for example, the bacterial cells are crushed by, for instance, pressurization treatment or ultrasonic treatment and then separated and purified in substantially the same manner as described above to obtain an enzyme of interest. Note that the above-mentioned series of steps (bacterial cell crushing, separation, and purification) may be carried out after the bacterial cells have been collected from culture broth by, for instance, filtration or centrifugation. The enzyme may be pulverized by a drying procedure such as lyophilization or drying under reduced pressure while a suitable excipient or drying aid may be used at that time.

As used herein, the activity of protein deamidase is measured by the following protocol.

(1) 0.1 ml aqueous solution containing a protein deamidase is added to 1 ml of 0.2 M phosphate buffer (pH 6.5) containing 30 mM Z-Gln-Gly, and the mixture is incubated at 37°C for 10 min. Then, 1 ml of 0.4 M TCA solution is added to stop the reaction. Next, 0.1 ml aqueous solution containing a protein deamidase is added to a mixture obtained by adding 1 ml of 0.2M phosphate buffer (pH 6.5) containing 30 mM Z-Gln-Gly and 1 ml of 0.4 M TCA solution, and the resulting mixture is incubated at 37°C for 10 min to prepare a blank sample.

(2) For the solutions obtained in (1), the amount of ammonia generated by the reaction is measured using an Ammonia Test Wako (Wako Pure Chemical Industries, Ltd.). The ammonia concentration in the reaction solution is determined from a standard curve that represents the relationship between the ammonia concentration and the absorbance (at 630 nm) and has been prepared using an ammonia standard solution (ammonium chloride).

(3) The activity of protein deamidase is calculated, from the following formula, while the amount of enzyme producing 1 $\mu$mol ammonia per min is set as 1 unit.

$$\text{Enzyme activity (U/mL)} = \text{Ammonia concentration in a reaction solution}$$

$$(\text{mg/L}) \times (1/17.03) \times (\text{Volume of reaction solution/Volume of enzyme solution}) \times$$

$$(1/10) \times \text{Df}$$

wherein the volume of reaction solution is 2.1, the volume of enzyme solution is 0.1, and Df is a dilution factor of the enzyme solution; and the molecular weight of ammonia is 17.03.

The conditions for the treatment with a protein deamidase are not particularly limited as long as the treatment is effective in improving the dispersibility of nut milk. The optimum reaction conditions may be set by adjusting the reaction temperature, the reaction time, and/or the amount of enzyme added (enzyme concentration).

Although not limited to the above example, the reaction temperature may be set, for example, within a range of 2°C to 70°C, preferably within a range of 5°C to 60°C, and more preferably within a range of 15°C to 50°C. Similarly, the reaction time may be set, for example, within a range of 10 min to 7 days, preferably within a range of 30 min to 3 days, and more preferably within a range of 1 h to 1 day. The amount of enzyme added may be set, for example, within a range of 0.01 (U/g protein) to 500 (U/g protein), preferably within a range of 0.02 (U/g protein) to 50 (U/g protein), and more preferably within a range of 0.2 (U/g protein) to 5 (U/g protein). Here, the "U/g protein" refers to the number of units per substrate nut protein (g). Note that as mentioned above, the protein concentration in untreated nut milk is not particularly limited, and untreated nut milk having a protein concentration of, for example, 0.2% (w/v) to 10.0% (w/v), preferably 0.2% (w/v) to 8.0% (w/v), and more preferably 0.2% (w/v) to 5.0% (w/v) is treated with a protein deamidase.

Here, when the conditions of the treatment with a protein deamidase are set, the following instructions (a) to (c) may be followed.

(a) When the reaction temperature is lowered, the reaction time is extended or the amount of enzyme added is increased (or both).

(b) When the reaction time is shortened, the reaction temperature is raised (provided that the temperature is not higher than 70°C and preferably 60°C or lower) or the amount of enzyme added is increased (or both).

(c) When the amount of enzyme added is decreased, the reaction temperature is raised (provided that the temperature is not higher than 70°C and preferably 60°C or lower) or the reaction time is extended (or both).

More specific instructions for setting the treatment condition are exemplified below.

In the case of 5°C ≤ the reaction temperature < 15°C, set the reaction time to more than 8 h (preferably 24 h or longer), or set the amount of enzyme added to 0.2 (U/g protein) or larger (preferably 1 (U/g protein) or larger).

In the case of 15°C ≤ reaction temperature < 25°C, set the reaction time to more than 7 h, or set the amount of enzyme added to more than 0.2 (U/g protein) (preferably 1 (U/g protein) or larger).

In the case of 25°C ≤ the reaction temperature < 40°C, set the reaction time to more than 5 h (preferably 7 h or longer), or set the amount of enzyme added to 0.2 (U/g protein) or larger (preferably 1 (U/g protein) or larger).

In the case of 40°C ≤ the reaction temperature < 50°C, set the reaction time to preferably 3 h or longer, or set the amount of enzyme added to preferably 0.2 (U/g protein) or larger.

In the case of 50 ≤ the reaction temperature (provided that the temperature is not higher than 70°C and preferably 60°C or lower), set the reaction time to preferably 3 h or longer, or set the amount of enzyme added to preferably 0.2 (U/g protein) or larger.

As described above, the nut milk of the invention excels in dispersibility, and is unlikely to cause protein aggregation. Typically, no protein aggregation occurs when the nut milk is mixed (added) to a weakly acidic (3 ≤ pH < 6) to weakly alkaline (8 ≤ pH < 11) liquid (provided that the pH of mixture is 5 or higher). The pH that causes no protein aggregation and is of the liquid after mixing is, for example, from 5 to 10, preferably from 5 to 9, and more preferably from 5 to 7. The liquid (beverage or liquid food) with which the nut milk of the invention is mixed is not particularly limited, and examples thereof include coffee, coffee beverages, and tea (e.g., black tea, green tea, oolong tea; reduced extract, reduced processed (e.g., concentrated, lyophilized) extract), tea beverages (e.g., flavored tea, milk tea, a tea beverage containing fruit juice), fruit juice, fruit juice beverages, sports drinks, nutritional drinks (e.g., protein drinks, care-use nutritional drinks), soups (Bouillon-based soup, stew, chowder, borsch, vegetable soup (e.g., tomato soup, corn soup, potage, pumpkin soup), miso-soup), curry, cocoa beverages, or chocolate beverages.

In a preferable embodiment of the invention, the plant milk has the features of excellent dispersibility and less occurrence of protein aggregation and is free of, for instance, an emulsifier (e.g., glycerin fatty acid ester, sucrose fatty acid ester, lecithin, saponin), a polysaccharide thickener (e.g., pectin, carboxymethyl cellulose), or a salt (e.g., a sea salt, a calcium salt, a phosphate) for preventing aggregation. In particular, the plant milk is free of any emulsifier or any polysaccharide thickener. As described above, the invention provides nut milk that meets the needs of consumers for products with little or no additives. Note that even in the preferable embodiment, use of an additive(s) for purposes (specifically, for example, adjustment of taste and flavor) other than prevention of aggregation is not prevented.

As can be seen from the above description, the nut milk of the invention can be produced by treating untreated nut milk with a protein deamidase. Thus, the nut milk of the invention is typically obtained by a production process including the following steps (1) and (2) of:

(1) providing nut milk; and
(2) treating the nut milk provided in step (1) with a protein deamidase.

Step (2), namely treatment with a protein deamidase, may be performed either before or after heat sterilization of nut milk. However, in order to simplify the production procedure, the step may be performed before heat sterilization of the nut milk, and a heat sterilization step that also serves as inactivation of the protein deamidase may then be performed (in other words, step (2) may be incorporated into the step of producing nut milk). Thus, in a preferable embodiment, step (2) is followed by "step (3) of performing heat treatment". The conditions for the heat treatment are not particularly limited as long as the protein deamidase can be inactivated and the nut milk can be sterilized. For example, the treatment is performed at a temperature of 70°C to 150°C for 1 sec to 5 h.

2. Use of Nut Milk

A second aspect of the present invention relates to use of nut milk of the invention. The nut milk of the invention excels in dispersibility, and is unlikely to cause protein aggregation. This feature is thus suitable for use in various beverages and/or liquid foods. Specifically, various beverages or various liquid foods blended with the nut milk of the invention are provided.

As shown in Examples described later, the detailed studies of the present inventors have revealed that (i) treatment with a protein deamidase can widen, to the acidic side, a pH range in which no aggregation occurs, and (ii) protein aggregation occurring when nut milk is mixed with, for instance, a beverage or liquid food depends on the pH of the beverage or the like after mixing with the nut milk, and when the pH is 5 or higher, no protein aggregation occurs. In view of this finding, the pH of the beverage or liquid food blended with the nut milk of the invention is preferably 5 or higher. More specifically, the pH of the beverage or liquid food blended with the nut milk of the invention is preferably from 5 to 9, more preferably from 5 to 8, and still more preferably from 5 to 7.5.

Examples of the beverage or liquid food include coffee beverages, coffee whiteners (e.g., use for other than coffee, such as black tea, is also assumed), tea beverages (e.g., flavored tea, milk tea, fruit juice-containing tea beverages), fruit juice beverages, sports drinks, nutritional drinks (e.g., protein drinks, care-use nutritional drinks), various soups, curry, cocoa beverages, or chocolate beverages. As can be seen from this example, the invention can be used not only for neutral beverages and/or liquid foods but also for weakly acidic beverages and/or liquid foods.

The nut milk is mixed with, for instance, other raw materials in the middle of the production procedure of the beverage or liquid food. Preferably, the nut milk is mixed at the final stage of the production procedure, i.e., after the other raw materials have been mixed and processed (at the stage of having a product form/shape). Provided that the mixing may be followed by sterilization treatment, addition of, for instance, seasonings, preservatives, flavors, and/or antioxidants in order to adjust the taste and/or maintain the quality, and so on. Meanwhile, in a preferable embodiment, the nut milk is mixed with the beverage or liquid food after the production procedure has been completed (i.e., in the form of not an intermediate product but a final product). In the case of this embodiment, the invention can be applied without changing the procedure for producing the beverage or liquid food.

EXAMPLES

1. To Prevent Aggregation in Coffee

To 100 mL of commercially available almond milk (manufactured by Rude Health; protein content: 1.5%; raw materials: almonds and water) was added a protein glutaminase "Amano" 500 (manufactured by Amano Enzyme Inc.; 500 U/g) in an amount of 1 U per g of proteins in the almond milk, and a reaction (deamidation reaction) was carried out at 50°C for 5 h. The enzyme was heat-inactivated by treatment at 95°C for 20 min, and the reaction solution was then cooled to 5°C to give enzyme-treated almond milk.
Commercially available instant coffee was dissolved in hot water to prepare a coffee liquid (2%). When 20 to 30 mL of the enzyme-treated almond milk was added to 150 mL of the coffee liquid (the pH after addition of the enzyme-treated almond milk was 5.7), no aggregation was observed. By contrast, evident aggregation was observed when non-enzyme-treated almond milk was used. Note that an experiment was conducted under the same conditions using peanut milk instead of almond milk, and the results were similar (no aggregation occurred in the case of enzyme-treated peanut milk).

2. Relationship between Protein Concentration of Nut Milk and Aggregation/Aggregation Preventing Effect

<Without Enzyme Treatment>

(1) Protocol

Commercially available almond milk (manufactured by Rude Health; protein content: 1.5%; raw materials: almonds and water) was diluted with tap water so as to have a protein concentration of 0.1, 0.5, or 1.5% (w/v), and then cooled to 5°C. Next, 5 mL of each was added to 50 mL of coffee liquid heated to 90°C to check the presence or absence of aggregation.

(2) Results

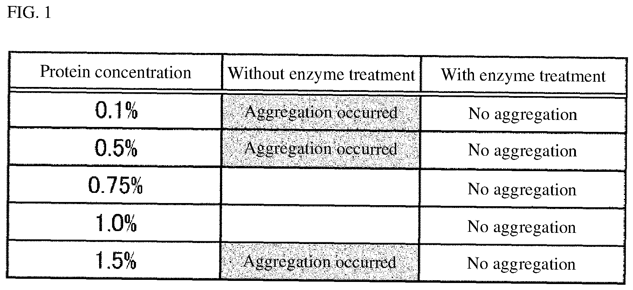

Aggregation in almond milk at any protein concentration was observed (Fig. 1).

<With Enzyme Treatment>

(1) Protocol

To commercially available almond milk (manufactured by Rude Health; protein content: 1.5%; raw materials: almonds and water) was added a protein glutaminase "Amano" 500 (manufactured by Amano Enzyme Inc.; 500 U/g) in an amount of 1 U per g of proteins in the almond milk, and a reaction (deamidation reaction) was carried out at 50°C for 5 h. The enzyme was heat-inactivated by treatment at 90°C for 15 min to give enzyme-treated almond milk. The enzyme-treated almond milk was diluted with tap water to have a protein concentration of 0.1, 0.5, 0.75, 1.0, or 1.5% (w/v), and then cooled to 5°C. Next, 5 ml of each was added to 50 mL of coffee liquid heated to 90°C to check the presence or absence of aggregation.

(2) Results

No aggregation was observed in the case of the almond milk at any protein concentration (Fig. 1).

3. Relationship between pH of Liquid and Aggregation/Aggregation Preventing Effect

(1) Protocol

The pH was adjusted with hydrochloric acid or sodium hydroxide, and 15 to 20 mL of non-enzyme-treated almond milk or enzyme-treated almond milk (at a protein concentration of 1.5% (w/v)) was then added to hot water heated to 90°C to check aggregation. Note that the enzyme-treated almond milk was prepared by the protocol described in the experiment of the above section 2.

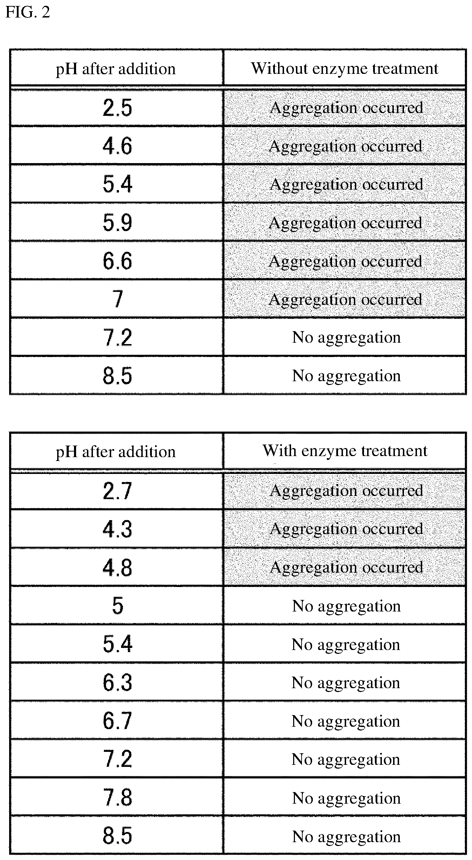

(2) Results (Fig. 2)

In the case of the non-enzyme-treated almond milk, aggregation was observed when the mixture after addition had a pH of from 2.5 to 7.0. By contrast, in the case of the enzyme-treated almond milk, aggregation was observed when the mixture after addition had a pH of from 2.7 to 4.8.

4. Effect of Preventing Aggregation in Various Liquids

4-1. Black Tea

(1) Protocol

Boiling water was poured onto a commercially available black tea pack (English Breakfast, manufactured by Twining and Company Limited); the tea was extracted for 2 to 3 min; and the tea pack was then taken out to prepare black tea. Non-enzyme-treated almond milk or enzyme-treated almond milk (at a protein concentration of 1.5% (w/v)) was added to this black tea, and the presence or absence of aggregation was then checked. The black tea immediately before addition of the almond milk had a temperature of 80°C and a pH of 5.2. The pH of the black tea after addition of the almond milk was 5.9. Note that the enzyme-treated almond milk was prepared by the protocol described in the experiment of the above section 2.

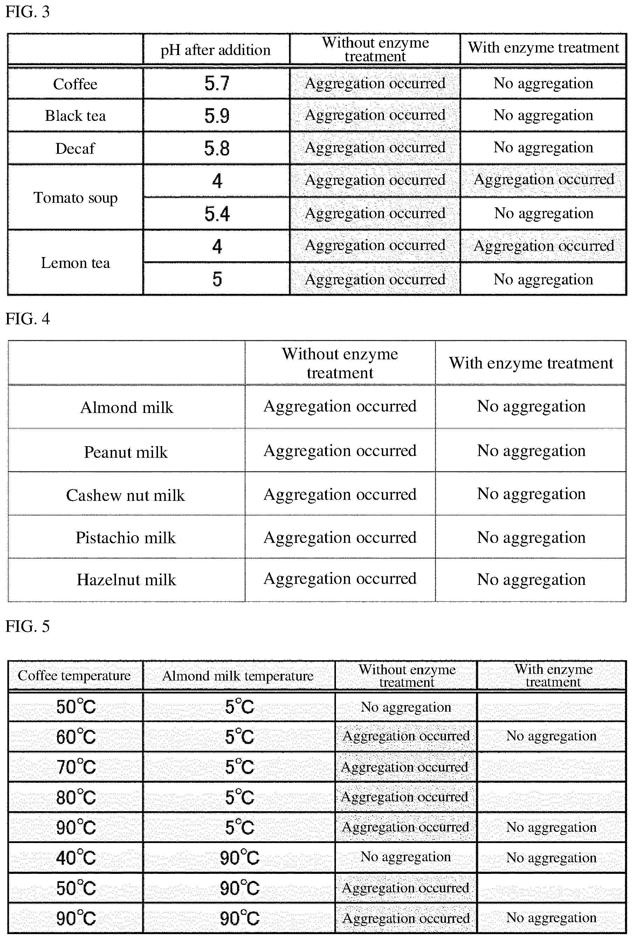

(2) Results (Fig. 3)

In the case of the non-enzyme-treated almond milk, slight aggregation was observed. By contrast, no aggregation was observed in the case of the enzyme-treated almond milk.

4-2.Lemon Tea

(1) Protocol

Boiling water was poured onto a commercially available black tea pack (English Breakfast, manufactured by Twining and Company Limited); the tea was extracted for 2 to 3 min; and the tea pack was then taken out to prepare black tea. After lemon juice was added and the pH was so adjusted, non-enzyme-treated almond milk or enzyme-treated almond milk (at a protein concentration of 1.5% (w/v)) was added to this black tea. Then, the presence or absence of aggregation was checked. The black tea immediately before addition of the almond milk had a temperature of 70°C. Note that the enzyme-treated almond milk was prepared by the protocol described in the experiment of the above section 2.

(2) Results (Fig. 3)

When the pH before addition of the almond milk was 3.5, aggregation was observed in the cases of both the non-enzyme-treated almond milk and the enzyme-treated almond milk. Note that the pH of the black tea after addition of the non-enzyme-treated almond milk was 3.9, and the pH of the black tea after addition of the enzyme-treated almond milk was 4.1.
When the pH before addition of the almond milk was 4.0, aggregation was observed in the case of the non-enzyme-treated almond milk. However, no aggregation was observed in case of the enzyme-treated almond milk. The pH of the black tea after addition of the non-enzyme-treated almond milk was 4.9, and the pH of the black tea after addition of the enzyme-treated almond milk was 5.0.

4-3. Decaf

(1) Protocol

Boiling water was poured onto commercially available decaf coffee powder (Nescafe Gold, manufactured by Nestlé Ltd.) to dissolve the powder well and prepare a decaf coffee liquid. Non-enzyme-treated almond milk or enzyme-treated almond milk (at a protein concentration of 1.5% (w/v)) was added to this liquid, and the presence or absence of aggregation was checked. The decaf coffee liquid immediately before addition of the almond milk had a temperature of 80°C and a pH of 5.3. In addition, the pH of the decaf coffee liquid after addition of the almond milk was 5.8. Note that the enzyme-treated almond milk was prepared by the protocol described in the experiment of the above section 2.

(2) Results (Fig. 3)

While aggregation was observed in the case of the non-enzyme-treated almond milk, no aggregation was observed in the case of the enzyme-treated almond milk.

4-4. Tomato Soup

(1) Protocol

A predetermined volume of boiling water was poured into commercially available chicken soup stock (Knorr Chicken Cube, manufactured by Unilever PLC) to completely dissolve the stock and prepare a chicken soup. Commercially available tomato puree was then added. After the quantity of puree added was changed and the pH pf the tomato soup was adjusted, non-enzyme-treated almond milk or enzyme-treated almond milk (at a protein concentration of 1.5% (w/v)) was added. The presence or absence of aggregation was then checked. The tomato soup immediately before addition of the almond milk was at 80°C. Note that the enzyme-treated almond milk was prepared by the protocol described in the experiment of the above section 2.

(2) Results (Fig. 3)

When the pH before addition of the almond milk was 5.0, aggregation was observed in the case of the non-enzyme-treated almond milk. However, no aggregation was observed in case of the enzyme-treated almond milk. Note that the pH of the tomato soup after addition of the non-enzyme-treated almond milk and the pH of the tomato soup after addition of the enzyme-treated almond milk were both 5.4.

When the pH before addition of the almond milk was 4.0, aggregation was observed in the cases of both the non-enzyme-treated almond milk and the enzyme-treated almond milk. Note that the pH of the tomato soup after addition of the non-enzyme-treated almond milk and the pH of the tomato soup after addition of the enzyme-treated almond milk were both 4.0. Note that a pH modifier citric acid added to the tomato puree has a strong buffering capacity. This may cause aggregation because the pH was unchanged even after addition of the almond milk.

5. Aggregation Preventing Effect in Cases of Nut Milk Other Than Almond Milk

(1) Protocol

To commercially available peanut milk (manufactured by Rude Health; protein content: 2.0%; raw materials: peanuts and water), commercially available cashew nut milk (manufactured by PLENISH, Inc.; protein content: 0.9%; raw materials: water, cashew nuts, and a salt), pistachio milk (manufactured by Borna Food Limited; protein content: 1.0%), or hazelnut milk (manufactured by Plenish; protein content: 0.6%) was added a protein glutaminase "Amano" 500 (manufactured by Amano Enzyme Inc.; 500 U/g) in an amount of 1 U per g of nut proteins. Then, the reaction (deamidation reaction) was carried out at 50°C for 5 h. After the enzymatic reaction, the enzyme was rapidly inactivated by treatment at 90°C for 15 min. The mixture was cooled in running water, and then cooled to 5°C in a refrigerator. After that, 5 mL of each was added to 50 mL of coffee liquid heated to 90°C to check the presence or absence of aggregation.

(2) Results (Fig. 4)

Any of peanut milk, cashew nut milk, pistachio milk, or hazelnut milk without enzyme treatment was found to cause aggregation. However, no aggregation was observed in the case of enzyme treatment. This result indicates that the enzyme treatment can exert substantially the same effects on nut milk other than the almond milk.

6. Relationship between Temperature of Liquid and Aggregation/Aggregation Preventing Effect

<Coffee Temperature Changed (Almond Milk Kept at 5°C)>

(1) Protocol

First, 5 mL of non-enzyme-treated almond milk or enzyme-treated almond milk cooled to 5°C was added to 50 mL of coffee adjusted at each temperature, and the presence or absence of aggregation was then checked.

(2) Results (Fig. 5)

In the case of the non-enzyme-treated almond milk, aggregation was observed at a coffee temperature of 60°C or higher. As the temperature became higher, the aggregation amount increased. By contrast, in the case of the enzyme-treated almond milk, aggregation preventing effect was observed (at a coffee temperature of 60°C and 90°C).

<Coffee Temperature Changed (Almond Milk Kept at 90°C)>

(1) Protocol

First, 5 mL of non-enzyme-treated almond milk or enzyme-treated almond milk heated to 90°C was added to 50 mL of coffee adjusted at each temperature, and the presence or absence of aggregation was checked.

(2) Results (Fig. 5)

When the non-enzyme-treated almond milk at 90°C was added to coffee at 90°C, aggregation was observed. In addition, when the non-enzyme-treated almond milk at 90°C was added to coffee at 50°C, aggregation was observed. However, when the non-enzyme-treated almond milk at 90°C was added to coffee at 40°C, no aggregation was observed. The aggregation may occur because when the non-enzyme-treated almond milk at 90°C was added to coffee at 50°C, the temperature of the coffee was temporarily increased. Besides, it seems that a higher post-mixing temperature (50°C or higher) is likely to cause aggregation.
No aggregation was observed when the enzyme-treated almond milk was added (to coffee at 40°C or 90°C).

7. To Investigate Enzyme Treatment Conditions (Amount of Enzyme Added, Reaction Temperature, and Reaction Time)

(1) Protocol

To commercially available almond milk (manufactured by Rude Health; protein content: 1.5%, raw material: almonds and water) was added a protein glutaminase "Amano" in an amount of 0.2 U, 1 U, or 5 U per g of proteins in the almond milk. A reaction (deamidation reaction) was then carried out at a given temperature (5°C, 15°C, 25°C, 40°C, or 50°C) for 3 to 24 h. After the enzymatic reaction, the enzyme was rapidly inactivated by treatment at 90°C for 15 min. The mixture was cooled in running water, and then cooled to 5°C in a refrigerator. After that, 5 mL of each was added to 50 mL of coffee liquid heated to 90°C to check the presence or absence of aggregation.

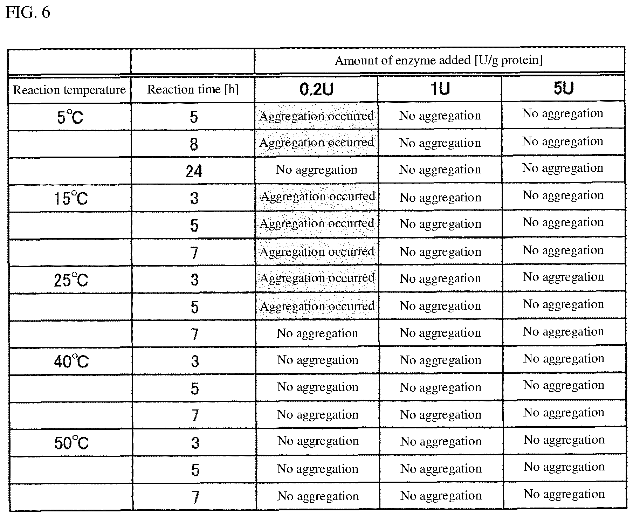

(2) Results (Fig. 6)

The effects vary depending on the amount of enzyme added, the reaction temperature, and/or the reaction time, but it has been found that aggregation can be prevented by adjusting these conditions. Specifically, in the case of a lower reaction temperature, a desired effect can be obtained by increasing the amount of enzyme added or extending the reaction time (or both). For instance, even when the reaction temperature is 5°C, aggregation can be effectively prevented in the case of the amount of enzyme added at 1 U or larger or in the case of a long reaction time. By contrast, in the case of a short reaction time, a desired effect can be obtained by raising the reaction temperature or increasing the amount of enzyme added (or both). For instance, even when the reaction time is 3 h, the aggregation preventing effect can be obtained in the case of the reaction temperature set to 40°C or higher or in the case of the amount of enzyme added at 1 U or larger. In addition, a higher reaction temperature or a longer reaction time (or both) makes it possible to decrease the amount of enzyme added. For instance, if the reaction temperature is 25°C or higher or the reaction time is prolonged, the amount of enzyme added can be 0.2 U or less.

<Summary>

- In the range of a nut protein concentration of from 0.1 to 1.5% (w/v), substantially the same aggregation preventing effect was observed regardless of the concentration. That is, it has been demonstrated that the enzyme treatment with a protein deamidase is effective in preventing aggregation in nut milk with various protein concentrations, and has general utility.
- Depending on the type of liquid to be mixed with nut milk, the tendency is that aggregation occurs when the pH is 7 or lower after the liquid is mixed with nut milk without enzyme treatment with a protein deamidase. However, in the case of the enzyme treatment, it has been found that the lower limit of aggregation can be extended to pH 5. It has been demonstrated that if the pH of liquid after mixed with nut milk is 5 or higher, the nut milk can be used for acidic liquid foods such as milk soups having a sour taste in addition to beverages such as coffee and black tea. Further, when the pH of liquid after mixed with milk is 5 or higher, milk lemon tea, the preparation of which is difficult even with cow milk, can be prepared. Thus, the invention is also applicable to various beverages and/or liquid foods using fruits having a sour taste.
- Basically, the pH of liquid to be mixed with nut milk significantly affects the aggregation. Then, as the temperature of the liquid becomes higher, the aggregation occurs more readily.
- The effects vary depending on the amount of enzyme added (enzyme concentration), the reaction temperature, and/or the reaction time.
- Not only almond milk but also peanut milk, cashew nut milk, pistachio milk, or hazelnut milk exhibited the same effects. Collectively, the enzyme treatment with a protein deamidase should be effective in preventing aggregation in nut milk in general.

INDUSTRIAL APPLICABILITY

The invention provides nut milk having excellent dispersibility without using any additive such as an emulsifier. This increased dispersibility enhances the value of nut milk itself and a beverage or liquid food using the nut milk. In addition, it is possible to provide a novel beverage or liquid food that cannot be realized conventionally.

Nut milk provided by the invention is not limited to existing uses, and should be utilized or applied to various uses (in particular, acidic beverages and acidic liquid foods). It is a great advantage of the invention that any additive(s) such as an emulsifier is dispensable. In addition, even in a case where the nut milk is added as a substitute for cow milk, soy milk, or the like to, for instance, coffee, a special operation for preventing aggregation is not required. This can increase convenience of consumers.

The invention is not limited to the description of the embodiments and examples according to the invention. Various modifications that can be easily conceived by those skilled in the art without departing from the scope of the Claims are also included in the invention. The contents of, for instance, the research articles, patent application publications, and patent publications disclosed herein are incorporated by reference in their entirety.

Claims

1. Nut milk having been treated with a protein deamidase.

2. The nut milk according to claim 1, wherein a raw material nut is one or two or more nuts selected from almonds, cashew nuts, hazelnuts, pecan nuts, macadamia nuts, pistachios, walnuts, Brazil nuts, peanuts, coconuts, chestnuts, sesames, and pine nuts.

3. The nut milk according to claim 1 or 2, wherein a nut protein concentration thereof is from 0.2% (w/v) to 10.0% (w/v).

4. The nut milk according to any one of claims 1 to 3, wherein dispersibility thereof is improved by the treatment.

5. The nut milk according to claim 4, wherein no protein aggregation occurs in a case of being mixed with a weakly acidic to weakly alkaline liquid, provided that a pH of mixture is 5 or higher.

6. The nut milk according to claim 5, wherein the liquid has a pH of from 5 to 7.

7. The nut milk according to claim 5, wherein the liquid is a beverage or liquid food selected from the group consisting of coffee, coffee beverages, tea, tea beverages, fruit juice, fruit juice beverages, sports drinks, nutritional drinks, soup, curry, cocoa beverages, and chocolate beverages.

8. The nut milk according to any one of claims 1 to 7, wherein no emulsifier or polysaccharide thickener for preventing aggregation is included.

9. The nut milk according to any one of claims 1 to 8, wherein the protein deamidase is an enzyme derived from a *Chryseobacterium* microorganism.

10. The nut milk according to claim 9, wherein the *Chryseobacterium* microorganism is *Chryseobacterium proteolyticum*.

11. A method for producing nut milk having improved dispersibility, comprising treating nut milk with a protein deamidase.

12. The method according to claim 11, comprising the following steps (1) and (2) of:

(1) providing nut milk; and
(2) treating the nut milk provided in step (1) with a protein deamidase.

13. The method according to claim 12, wherein the nut milk in step (1) is nut milk before heat sterilization.

14. The method according to claim 13, further comprising the following step (3) of:
(3) performing heat treatment.

15. A beverage or liquid food comprising the nut milk according to any one of claims 1 to 10.

16. The beverage or liquid food according to claim 15, wherein the beverage or liquid food has a pH of 5 or higher.

17. The beverage or liquid food according to claim 15, wherein the beverage or liquid food is selected from the group consisting of coffee beverages, coffee whiteners, tea beverages, fruit juice beverages, sports drinks, nutritional drinks, soups, curry, cocoa beverages, and chocolate beverages.

FIG. 1

| Protein concentration | Without enzyme treatment | With enzyme treatment |
|---|---|---|
| 0.1% | Aggregation occurred | No aggregation |
| 0.5% | Aggregation occurred | No aggregation |
| 0.75% | | No aggregation |
| 1.0% | | No aggregation |
| 1.5% | Aggregation occurred | No aggregation |

FIG. 2

| pH after addition | Without enzyme treatment |
|:-:|:-:|
| 2.5 | Aggregation occurred |
| 4.6 | Aggregation occurred |
| 5.4 | Aggregation occurred |
| 5.9 | Aggregation occurred |
| 6.6 | Aggregation occurred |
| 7 | Aggregation occurred |
| 7.2 | No aggregation |
| 8.5 | No aggregation |

| pH after addition | With enzyme treatment |
|:-:|:-:|
| 2.7 | Aggregation occurred |
| 4.3 | Aggregation occurred |
| 4.8 | Aggregation occurred |
| 5 | No aggregation |
| 5.4 | No aggregation |
| 6.3 | No aggregation |
| 6.7 | No aggregation |
| 7.2 | No aggregation |
| 7.8 | No aggregation |
| 8.5 | No aggregation |

FIG. 3

| | pH after addition | Without enzyme treatment | With enzyme treatment |
|---|---|---|---|
| Coffee | 5.7 | Aggregation occurred | No aggregation |
| Black tea | 5.9 | Aggregation occurred | No aggregation |
| Decaf | 5.8 | Aggregation occurred | No aggregation |
| Tomato soup | 4 | Aggregation occurred | Aggregation occurred |
| | 5.4 | Aggregation occurred | No aggregation |
| Lemon tea | 4 | Aggregation occurred | Aggregation occurred |
| | 5 | Aggregation occurred | No aggregation |

FIG. 4

| | Without enzyme treatment | With enzyme treatment |
|---|---|---|
| Almond milk | Aggregation occurred | No aggregation |
| Peanut milk | Aggregation occurred | No aggregation |
| Cashew nut milk | Aggregation occurred | No aggregation |
| Pistachio milk | Aggregation occurred | No aggregation |
| Hazelnut milk | Aggregation occurred | No aggregation |

FIG. 5

| Coffee temperature | Almond milk temperature | Without enzyme treatment | With enzyme treatment |
|---|---|---|---|
| 50°C | 5°C | No aggregation | |
| 60°C | 5°C | Aggregation occurred | No aggregation |
| 70°C | 5°C | Aggregation occurred | |
| 80°C | 5°C | Aggregation occurred | |
| 90°C | 5°C | Aggregation occurred | No aggregation |
| 40°C | 90°C | No aggregation | No aggregation |
| 50°C | 90°C | Aggregation occurred | |
| 90°C | 90°C | Aggregation occurred | No aggregation |

FIG. 6

| | | Amount of enzyme added [U/g protein] | | |
|---|---|---|---|---|
| Reaction temperature | Reaction time [h] | 0.2U | 1U | 5U |
| 5°C | 5 | Aggregation occurred | No aggregation | No aggregation |
| | 8 | Aggregation occurred | No aggregation | No aggregation |
| | 24 | No aggregation | No aggregation | No aggregation |
| 15°C | 3 | Aggregation occurred | No aggregation | No aggregation |
| | 5 | Aggregation occurred | No aggregation | No aggregation |
| | 7 | Aggregation occurred | No aggregation | No aggregation |
| 25°C | 3 | Aggregation occurred | No aggregation | No aggregation |
| | 5 | Aggregation occurred | No aggregation | No aggregation |
| | 7 | No aggregation | No aggregation | No aggregation |
| 40°C | 3 | No aggregation | No aggregation | No aggregation |
| | 5 | No aggregation | No aggregation | No aggregation |
| | 7 | No aggregation | No aggregation | No aggregation |
| 50°C | 3 | No aggregation | No aggregation | No aggregation |
| | 5 | No aggregation | No aggregation | No aggregation |
| | 7 | No aggregation | No aggregation | No aggregation |

## INTERNATIONAL SEARCH REPORT

| International application No. |
|---|
| PCT/JP2020/006432 |

**A. CLASSIFICATION OF SUBJECT MATTER**
A23C 11/10(2006.01)i; A23L 25/00(2016.01)i; C12N 9/10(2006.01)i
FI: A23L25/00; A23C1 1/10; C12N9/10
According to International Patent Classification (IPC) or to both national classification and IPC

**B. FIELDS SEARCHED**

Minimum documentation searched (classification system followed by classification symbols)
A23C11/10; A23L25/00; C12N9/10

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN), FSTA (STN)

**C. DOCUMENTS CONSIDERED TO BE RELEVANT**

| Category* | Citation of document, with indication, where appropriate, of the relevant passages | Relevant to claim No. |
|---|---|---|
| Y | SUPPAVORASATIT, Inthawoot, LEE, Soo-Yeun and CADWALLADER, Keith R., "Effect of Enzymatic Protein Deamidation on Protein Solubility and Flavor Binding Properties of Soymilk", J. Food Sci., 2013, vol. 78, no. 1, pp. C1-7 summary, fig. 1 | 1-17 |
| Y | YAMAGUCHI, Shotaro and YOKOE, Masaaki, "A novel protein-deamidating enzyme from Chryseobacterium proteolyticum sp. nov., a newly isolated bacterium from soil", Appl. Environ. Microbial., August 2000, vol. 66, no. 8, pp. 3337-3343, page 3337, left column, lines 5-16, page 3337, left column, line 1 to right column, line 8 | 1-17 |
| Y | SETHI, Swati, TYAGI, S. K. and ANURAG, Rahul K., "Plant-based milk alternatives an emerging segment of functional beverages: a review", J. Food Sci. Technol., September 2016, vol. 53, no. 9, pp. 3408-3423, summary, table 1 | 1-17 |
| A | JP 2017-516469 A (FRIESLANDCAMPINA NEDERLAND B.V.) 22.06.2017 (2017-06-22) claims 1-18, examples | 1-17 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03 April 2020 (03.04.2020) | 12 May 2020 (12.05.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| | | International application no. |
|---|---|---|
| | | PCT/JP2020/006432 |

| Patent Documents referred in the Report | Publication Date | Patent Family | Publication Date |
|---|---|---|---|
| JP 2017-516469 A | 22 Jun. 2017 | US 2017/0156375 A1 claims 19-38, examples | |

Form PCT/ISA/210 (patent family annex) (January 2015)

# EP 3 928 627 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019029904 A
- WO 2012176852 A
- JP 3885194 B
- WO 2011108633 A
- JP 2000050887 A
- JP 2001218590 A
- WO 2006075772 A